# EUROPEAN PATENT SPECIFICATION

(11) **EP 2 737 914 B1**
(45) Date of publication and mention of the grant of the patent: **14.10.2015**
(21) Application number: 12818039.5
(22) Date of filing: 24.07.2012
(51) Int. Cl.: A61M 1/00, B32B 3/26, A47G 27/02, A47L 23/26

(54) **COLLECTOR OF LIQUID**
FLÜSSIGKEITSKOLLEKTOR
COLLECTEUR DE LIQUIDE

(30) Priority: 28.07.2011 ES 201131300 P
(43) Date of publication of application: 04.06.2014
(73) Proprietor: Establecimientos Sumisan, S.A., 20009 San Sebastian (Guipúzcoa) (ES)
(72) Inventor: BUREAU, Maxime Pierrick François, 20009 San Sebastian (Guipúzcoa) (ES); ABREU DE CON, Iñigo, 20009 San Sebastian (Guipúzcoa) (ES)
(74) Representative: Ungria López, Javier
(86) International application number: PCT/ES2012/070561
(87) International publication number: WO 2013/014317

(56) References cited:
- EP-A1- 0 547 496
- WO-A1-2007/030103
- US-A- 5 176 667
- US-A- 5 827 246
- US-A1- 2005 133 092
- US-A1- 2006 015 080
- US-A1- 2006 041 238
- US-A1- 2006 286 334
- US-B1- 6 938 639
- US-B1- 7 131 965

## Description

### INVENTION PURPOSE

The present invention refers to a collector of liquid, preferably surgical liquid, with a multi-layered structure and a design and configuration which facilitate the effective and silent absorption of liquid which has been spilt on the floor and make it possible for people to step over the collector without the liquid spreading out of the collector.

### INVENTION BACKGROUND AND TECHNICAL PROBLEMS TO BE RESOLVED

In surgical operations, especially in endoscopic process in which a lot of serum is used, a large proportion of the liquids used in the operation end up on the floor mixed with patients' blood. Once the operation has ended, it is therefore necessary to collect this liquid before cleaning and disinfecting the surgery floor.

This collection and cleaning is time consuming, therefore systems to try to solve the root of the problem have been proposed, for example the use of pads or bags which minimise the liquid content falling to the floor whilst the surgical operation is carried out. However, they are not one hundred per cent effective when it comes to eliminating spillages of liquid on the floor.

Furthermore, there are various systems to collect and evacuate liquid which has been spilt on the floor. Documents WO 2008 153 545 A1, EP 0 547 496 B1 and EP 1 474 060 A1 show a flat structure with an absorbent upper layer and an area which catches the liquid in the lower part. In this area which catches the liquid, there is a suction tube used to evacuate the liquid collected.

The problem with these systems lies in their configuration and design which do not lead to the effective collection of liquid. Moreover, they are noisy owing to air being absorbed by the suction tube as well as liquid.

Many of these systems are also problematic or inconvenient because they cannot be placed in areas where people are working in the surgery, given that if somebody stood on one, the liquid absorbed by these systems would once again spread on the floor, as is the case with a wet sponge.

### INVENTION DESCRIPTION

With the aim of meeting goals and avoiding the abovementioned difficulties, the invention proposes a collector of liquid with a multi-layered structure whose layers are joined together by their edges using high frequency heat soldering.

This collector focuses fundamentally on eliminating noise from the suction part of the system, thus enabling the concentration of people carrying out work in an area close to the collector. It also focuses on the possibility of stepping on this collector, object of the invention, significantly improving mobility of the people working in close proximity of it.

The multi-layered collector of liquid comprises a first layer, which in turn comprises a set of openings and a third layer which comprises another set of openings. It also comprises a second layer, located above the first layer and below the third layer, comprising a vacuum tube and a material with structural consistency. This material enables a uniform vacuum area for liquid collection to be created and maintained at all times. Furthermore, it comprises a fourth layer, located immediately above the third layer, comprising a material that absorbs by capillarity.

A characteristic of the invention is that liquid coming from the first and third layers is evacuated via the vacuum tube of the second layer connected to a suction system serving to extract liquid. The first layer absorbs the liquid directly from the floor whilst liquid reaches the third layer after having been absorbed by and distributed uniformly on the fourth layer by the material that absorbs by capillarity.

Another characteristic of the collector of liquid, object of the invention, is that a fifth layer might also be included immediately above the fourth layer. This fifth layer would be made of one of the following non-woven materials: cellulose, polypropylene, a combination of cellulose and polypropylene, printed hydrophilic polyethylene wool, polyester and polyethylene. This fifth layer is capable of absorbing and passing to the fourth layer any amount of liquid which may be spilt on it without retaining it. This prevents liquid being splashed and forming puddles around the feet of those working in the area. Likewise, it prevents the fourth layer from tearing when people working in the area graze the collector of liquid with their shoes.

Given that this fifth layer does not absorb liquid, it prevents the collector of liquid from becoming too heavy to throw away easily and hygienically. However it does retain liquids for the necessary amount of time to allow the other layers to evacuate it via capillarity and vacuum, according to their distinct functions.

Another characteristic of the invention is that the set of openings in the first and third layer may be blocked off by a water-soluble material i.e. poly vinyl alcohol.

The first layer comprises at least one opening near to each of its corners and at least one opening in the middle which create a suction effect, thus allowing the first layer to stick to the floor. On the other hand, the set of openings of the third layer are arranged symmetrically along the entire surface and are more numerous than the set of openings of the first layer.

The first layer comprises either PVC (polyvinyl chloride), polyurethane or polyethylene. The second layer comprises one of the following coarse mesh materials: warped polyethylene sackcloth or multifilament polypropylene. The third layer comprises a PVC (polyvinyl chloride), polyurethane or polyethylene material. The fourth and fifth layers comprise a viscose sheet made of one of the following materials: cellulose, polypropylene, a combination of cellulose and polypropylene, printed hydrophilic polyethylene wool, polyester or polyethylene.

### DESCRIPTION OF THE DRAWINGS

In order to facilitate the easy understanding of the description herein, a series of drawings accompany the invention, serving as a non-limiting example of its use:
- Figure 1 is a cross section of the collector of liquids, object of the invention, illustrating the distribution of layers in the collector of liquids which comprises a vacuum tube, according to one of the embodiments.
- Figure 2 is a top elevation of a first layer of the collector.
- Figure 3 is a top elevation of a second layer with a detailed representation and within this detailed representation, a detailed representation of A-A.
- Figure 4 is a top elevation of a third layer of the collector.

Below is a list of the various elements represented in the figures which form part of the invention:
1 = First layer
2 = Second layer
3 = Third layer
4 = Fourth layer
5 = Fifth layer
6' = Opening of the first layer (1)
6'' = Opening of the third layer (3)
7 = Vacuum tube
8 = Soldering
9 = Floor

### DETAILED DESCRIPTION OF THE PREFERRED INVENTION

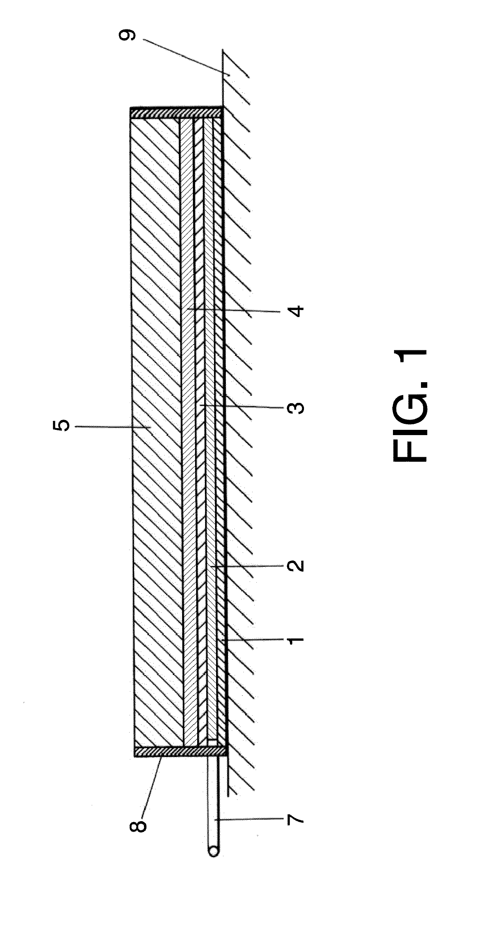

As already explained and further demonstrated in Figure 1, the collector of liquid, object of the invention, is determined from a series of layers being placed over one another, these layers being joined together at their edges by means of high-frequency heat-soldering (8), the aggregate having a total ply of 5 to 50 mm.

There is a fifth layer (5) in the upper part of the collector, situated immediately above the fourth layer (4) which is in turn situated immediately above the third layer (3) and so on and so forth until the first layer (1) is reached. A vacuum tube (5) can be seen in the second layer which is connected to a surgery suction system and used to create a vacuum whilst it absorbs liquid.

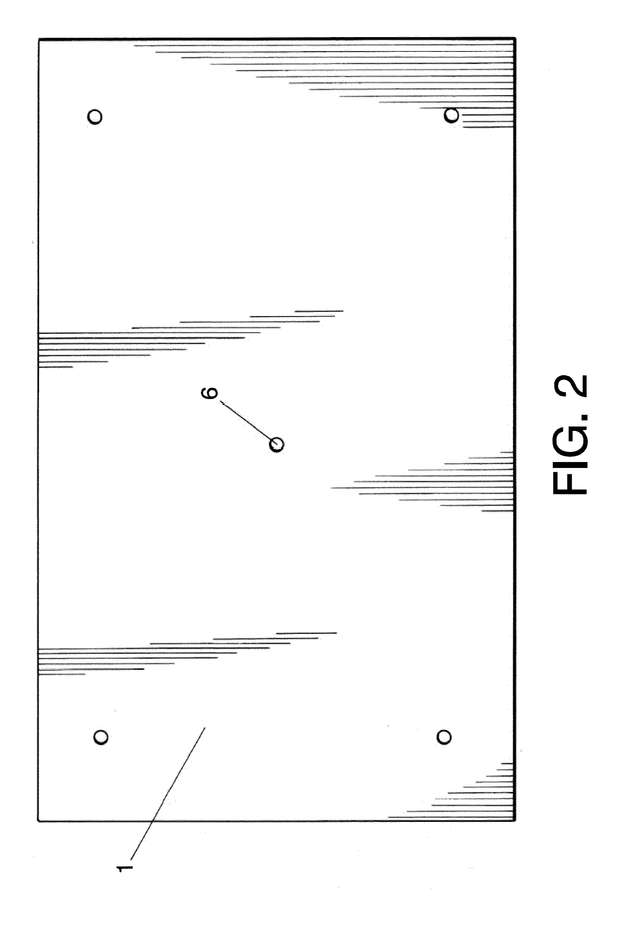

This first layer (1), made of PVC, polyurethane or polyethylene, as shown in Figure 2, is the layer which will contact the floor (9). This first layer (1) comprises a set of openings (6') blocked by a water-soluble material which prevents air from being absorbed instead of liquid. This eliminates the noisy stage at the beginning of surgical operations, especially in endoscopic processes during which a large amount of serum is used. This is because the suction process does not begin until the openings (6') have opened up, upon coming into contact with liquid (mainly serum but perhaps also blood) and also because the flow of liquid which previously came into contact with the collector, object of the invention, thereby absorbed by it, is great enough to generate continuous flow through the openings (6').

The set of openings (6') of the first layer (1) are distributed in such a way that at least one opening (6') is near to each of its corners and with at least one opening (6') in the central area. These openings (6') serve two purposes: absorbing liquids from the surgery floor (9) and creating a suction effect which enables the first layer (1) to stick to the floor (9) when liquid is not flowing through the openings (6'). However the first layer (1) comprises an anti-slip material which helps this layer (1) to remain stuck to a certain point thus preventing the staff stepping over the collector, object of the invention, from slipping.

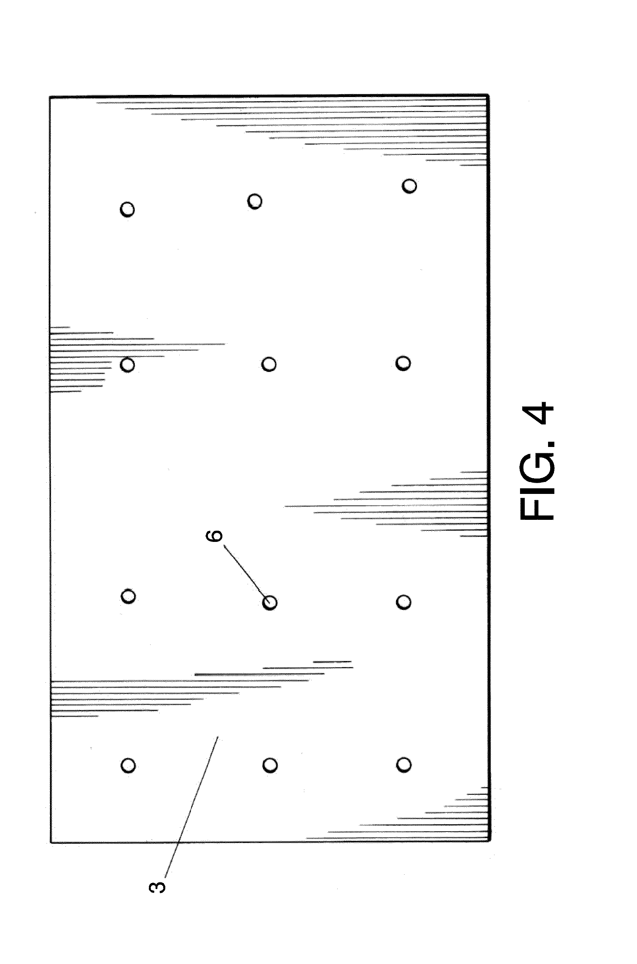

Moreover, the third layer (3) made of PVC, polyurethane or polyethylene and represented in Figure 4, also comprises a set of openings (6''). These openings (6'') have a similar diameter to those openings (6') of the first layer (1) but are greater in number and are arranged symmetrically along the length of the third layer (3). This set of openings constrict to allow liquid to flow through once the water-soluble material which was blocking them has dissolved in the same way as the set of openings (6') of the first layer (1). In this instance the liquid arrives descending from the upper part of the invention.

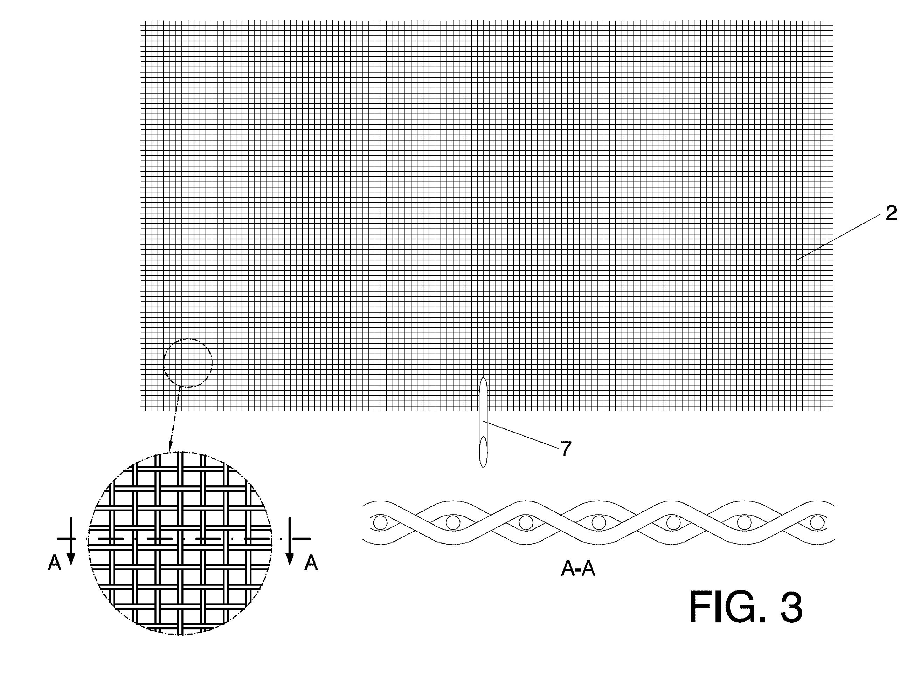

Figure 3 is a more detailed representation of the second layer (2) which serves to create a uniform vacuum area, thus preventing the first (1) and third (3) layers from collapsing when the vacuum is created by suction, i.e., preventing the first layer (1) to come into contact with the third layer (3).

In order to prevent the abovementioned vacuum collapse, the second layer (2) is made of warped polyethylene sackcloth or multifilament polypropylene mesh, which, alongside maintaining a permanent hollow inside the collector, makes it possible for the collector, object of the invention, to be stood on without running the risk of liquid suction becoming less effective, even at points far away from the vacuum connector. This vacuum connection consists of at least one vacuum tube (7) being inserted in the second layer (2).

The fourth layer (4) is a viscose sheet made of polypropylene and hydrophilic polyethylene wool which absorbs by capillarity. This fourth layer (4) serves to spread the liquid falling on in it along the length of the layer (4) thus improving the invention's suction efficiency.

The fifth layer (5) is a sheet of printed, high-density hydrophilic polyethylene wool and serves to prevent liquid from being splashed on the feet of those working above the collector and in the areas surrounding it when they step on the fifth layer (5). This layer (5) is highly absorbent and does not require the vacuum system to be working thus preventing the collector from forming puddles.

Now the nature of the invention has been described, its opportune effects will be stated though the use of the collector is not limited to this statement. Rather, opportune modifications may be introduced provided that they do not alter its essential characteristics.

## Claims

1. Collector of liquid comprising a multi-layered structure, **characterised in that** it comprises:
- a first layer (1) which comprises a set of openings (6') and a third layer (3) comprising another set of openings (6");
- a second layer (2) located above the first layer (1) and below the third layer (3), this second layer (2) comprising a vacuum tube (7) and a material whose structural consistency facilitates the generation and maintenance of a uniform vacuum area for liquid collection at all times; and
- a fourth layer (4) located immediately above the third layer (3), this fourth layer (4) comprising a material that absorbs by capillarity;
in such a way that liquid coming from the first (1) and third (3) layers is evacuated via the vacuum tube (7) located in the second layer (2), where the liquid arrives at this third layer (3) after having been absorbed by the fourth layer (4) and distributed uniformly along this fourth layer (4) by means of the material that absorbs by capillarity.

2. Collector of liquid according to claim 1, **characterised in that** it comprises:
- a fifth layer (5), located immediately above the fourth layer (4), which comprises one of the following non-woven absorbent materials: cellulose, polypropylene, a combination of cellulose and polypropylene, printed hydrophilic polyethylene wool, polyester or polyethylene.

3. Collector of liquid according to claim 1 or 2, **characterised in that** the vacuum tube (7) is connected to a suction system to extract liquid by means of suction.

4. Collector of liquid according to one of the preceding claims, **characterised in that** the set of openings (6') of the first layer (1) and the set of openings (6'') of the third layer (3), are blocked by a water-soluble solution.

5. Collector of liquid according to claim 4, **characterised in that** the water-soluble solution in the set of openings (6') of the first layer (1) and the set of openings (6'') of the third layer (3), is poly vinyl alcohol.

6. Collector of liquid according to one of the preceding claims, **characterised in that** the third layer (3) comprises a greater number of openings (6'') than the openings (6') located on the first layer (1).

7. Collector of liquid according to one of the preceding claims, **characterised in that** the first layer (1) comprises at least one opening (6') near to each one of its corners and at least one opening (6') in the central area of the first layer (1).

8. Collector of liquid according to one of claims 1 to 6, **characterised in that** the openings (6'') of the third layer (3) are arranged symmetrically along the whole of its (3) surface.

9. Collector of liquid according to claim 1 or 2, **characterised in that** the set of layers are joined together by their edges using high frequency heat soldering.

10. Collector of liquid according to claim 1 or 2, **characterised in that** the set of layers has a total ply of 5 to 50 mm.

11. Collector of liquid according to claim 1 or 2, **characterised in that** the first layer (1) comprises one material selected from PVC, polyurethane and polyethylene.

12. Collector of liquid according to claim 1 or 2, **characterised in that** the second layer (2) comprises one coarse mesh materials selected from warped polyethylene sackcloth and multifilament polypropylene.

13. Collector of liquid according to claim 1 or 2, **characterised in that** the third layer (3) comprises one material selected from PVC, polyurethane and polyethylene.

14. Collector of liquid according to claim 1 or 2, **characterised in that** the fourth layer (4) comprises a viscose sheet made of one material selected from cellulose, polypropylene, a combination of cellulose and polypropylene, printed hydrophilic polyethylene wool, polyester and polyethylene.

## Patentansprüche

1. Flüssigkeitskollektor umfassend eine Mehrschichtenstruktur, **dadurch gekennzeichnet, dass** er umfasst:
- eine erste Schicht (1), welche einen Satz von Öffnungen (6') umfasst, und eine dritte Schicht (3), die einen weiteren Satz von Öffnungen (6") umfasst;
- eine zweite Schicht (2), die sich über der ersten Schicht (1) und unter der dritten Schicht (3) befindet, wobei diese zweite Schicht (2) ein Vakuumrohr (7) und ein Material umfasst, dessen strukturelle Konsistenz jederzeit die Erzeugung und Aufrechterhaltung eines einheitlichen Vakuumbereichs für die Flüssigkeitserfassung ermöglicht; und
- eine vierte Schicht (4), die sich unmittelbar über der dritten Schicht (3) befindet, wobei diese vierte Schicht (4) ein Material umfasst, welches durch Kapillarwirkung absorbiert;
auf eine solche Weise, dass Flüssigkeit, die aus den ersten (1) und dritten (3) Schichten kommt, über das Vakuumrohr (7), das sich in der zweiten Schicht (2) befindet, abgeführt wird, wobei die Flüssigkeit an dieser dritten Schicht (3) ankommt, nachdem sie durch die vierte Schicht (4) absorbiert und entlang dieser vierten Schicht (4) mittels des Materials, welches durch Kapillarwirkung absorbiert, einheitlich verteilt worden ist.

2. Flüssigkeitskollektor nach Anspruch 1, **dadurch gekennzeichnet, dass** er umfasst:
- eine fünfte Schicht (5), die sich unmittelbar über der vierten Schicht (4) befindet, welche eines der folgenden absorbierenden Vliesstoffmaterialien umfasst: Cellulose, Polypropylen, eine Kombination von Cellulose und Polypropylen, gedruckte hydrophile Polyethylenwolle, Polyester oder Polyethylen.

3. Flüssigkeitskollektor nach Anspruch 1 oder 2, **dadurch gekennzeichnet, dass** das Vakuumrohr (7) mit einem Absaugsystem verbunden ist, um Flüssigkeit mittels Absaugung zu extrahieren.

4. Flüssigkeitskollektor nach einem der vorangehenden Ansprüche, **dadurch gekennzeichnet, dass** der Satz von Öffnungen (6') der ersten Schicht (1) und der Satz von Öffnungen (6") der dritten Schicht (3) durch eine wasserlösliche Lösung blockiert sind.

5. Flüssigkeitskollektor nach Anspruch 4, **dadurch gekennzeichnet, dass** die wasserlösliche Lösung in dem Satz von Öffnungen (6') der ersten Schicht (1) und dem Satz von Öffnungen (6") der dritten Schicht (3) Polyvinylalkohol ist.

6. Flüssigkeitskollektor nach einem der vorangehenden Ansprüche, **dadurch gekennzeichnet, dass** die dritte Schicht (3) eine größere Zahl von Öffnungen (6") als die Öffnungen (6'), die sich an der ersten Schicht (1) befinden, umfasst.

7. Flüssigkeitskollektor nach einem der vorangehenden Ansprüche, **dadurch gekennzeichnet, dass** die erste Schicht (1) wenigstens eine Öffnung (6') in der Nähe von jeder von ihren Ecken und wenigstens eine Öffnung (6') im zentralen Bereich der ersten Schicht (1) umfasst.

8. Flüssigkeitskollektor nach einem der Ansprüche 1 bis 6, **dadurch gekennzeichnet, dass** die Öffnungen (6") der dritten Schicht (3) symmetrisch entlang der Gesamtheit ihrer (3) Oberfläche angeordnet sind.

9. Flüssigkeitskollektor nach Anspruch 1 oder 2, **dadurch gekennzeichnet, dass** der Satz von Schichten mittels thermischen Hochfrequenzverschweißens an ihren Rändern miteinander verbunden sind.

10. Flüssigkeitskollektor nach Anspruch 1 oder 2, **dadurch gekennzeichnet, dass** der Satz von Schichten eine Gesamtdicke von 5 bis 50 mm aufweist.

11. Flüssigkeitskollektor nach Anspruch 1 oder 2, **dadurch gekennzeichnet, dass** die erste Schicht (1) ein Material, ausgewählt aus PVC, Polyurethan und Polyethylen, umfasst.

12. Flüssigkeitskollektor nach Anspruch 1 oder 2, **dadurch gekennzeichnet, dass** die zweite Schicht (2) ein grobes Maschenmaterial, ausgewählt aus geschärtem Polyethylen-Sackleinen und Multifilament-Polypropylen, umfasst.

13. Flüssigkeitskollektor nach Anspruch 1 oder 2, **dadurch gekennzeichnet, dass** die dritte Schicht (3) ein Material, ausgewählt aus PVC, Polyurethan und Polyethylen, umfasst.

14. Flüssigkeitskollektor nach Anspruch 1 oder 2, **dadurch gekennzeichnet, dass** die vierte Schicht (4) eine Viskoselage umfasst, die aus einem Material, ausgewählt aus Cellulose, Polypropylen, einer Kombination von Cellulose und Polypropylen, gedruckter hydrophiler Polyethylenwolle, Polyester und Polyethylen, gefertigt ist.

## Revendications

1. Collecteur de liquide comprenant une structure multicouche, **caractérisé en ce qu'**il comprend :
une première couche (1) qui comprend un ensemble d'ouvertures (6') et une troisième couche (3) comprenant un autre ensemble d'ouvertures (6") ;
une deuxième couche (2) positionnée au-dessus de la première couche (1) et au-dessous de la troisième couche (3), cette deuxième couche (2) comprenant un tube à vide (7) et un matériau dont la consistance structurelle facilite la génération et le maintien d'une zone de vide uniforme pour la collecte du liquide à tous moments ; et
une quatrième couche (4) positionnée immédiatement au-dessus de la troisième couche (3), cette quatrième couche (4) comprenant un matériau qui absorbe par capillarité ;
de sorte que le liquide provenant des première (1) et troisième (3) couches est évacué via le tube à vide (7) positionné dans la deuxième couche (2), où le liquide arrive au niveau de cette troisième couche (3) après avoir été absorbé par la quatrième couche (4) et distribué uniformément le long de cette quatrième couche (4) au moyen du matériau qui absorbe par capillarité.

2. Collecteur de liquide selon la revendication 1, **caractérisé en ce qu'**il comprend :
une cinquième couche (5) positionnée immédiatement au-dessus de la quatrième couche (4), qui comprend l'un des matériaux absorbants non tissés suivants : la cellulose, le polypropylène, une combinaison de cellulose et de polypropylène, la laine de polyéthylène hydrophile imprimée, le polyester ou le polyéthylène.

3. Collecteur de liquide selon la revendication 1 ou 2, **caractérisé en ce que** le tube à vide (7) est raccordé à un système d'aspiration pour extraire le liquide au moyen d'aspiration.

4. Collecteur de liquide selon l'une des revendications précédentes, **caractérisé en ce que** l'ensemble d'ouvertures (6') de la première couche (1) et l'ensemble d'ouvertures (6") de la troisième couche (3) sont bloqués par une solution soluble dans l'eau.

5. Collecteur de liquide selon la revendication 4, **caractérisé en ce que** la solution soluble dans l'eau dans l'ensemble d'ouvertures (6') de la première couche (1) et l'ensemble d'ouvertures (6") de la troisième couche (3), est de l'alcool polyvinylique.

6. Collecteur de liquide selon l'une des revendications précédentes, **caractérisé en ce que** la troisième couche (3) comprend un plus grand nombre d'ouvertures (6") que les ouvertures (6') positionnées sur la première couche (1).

7. Collecteur de liquide selon l'une des revendications précédentes, **caractérisé en ce que** la première couche (1) comprend au moins une ouverture (6') à proximité de chacun de ses coins et au moins une ouverture (6') dans la zone centrale de la première couche (1).

8. Collecteur de liquide selon l'une des revendications 1 à 6, **caractérisé en ce que** les ouvertures (6") de la troisième couche (3) sont agencées symétriquement le long de la totalité de sa surface (3).

9. Collecteur de liquide selon la revendication 1 ou 2, **caractérisé en ce que** l'ensemble des couches sont assemblées par leurs bords en utilisant le soudage par chauffage à haute fréquence.

10. Collecteur de liquide selon la revendication 1 ou 2, **caractérisé en ce que** l'ensemble des couches a une épaisseur totale de 5 à 50 mm.

11. Collecteur de liquide selon la revendication 1 ou 2, **caractérisé en ce que** la première couche (1) comprend un matériau choisi parmi le PVC, le polyuréthane et le polyéthylène.

12. Collecteur de liquide selon la revendication 1 ou 2, **caractérisé en ce que** la seconde couche (2) comprend un matériau à maille grossière choisi parmi la toile d'emballage gondolée en polyéthylène et le polypropylène multifilament.

13. Collecteur de liquide selon la revendication 1 ou 2, **caractérisé en ce que** la troisième couche (3) comprend un matériau choisi parmi le PVC, le polyuréthane et le polyéthylène.

14. Collecteur de liquide selon la revendication 1 ou 2, **caractérisé en ce que** la quatrième couche (4) comprend une feuille de viscose réalisée avec un matériau choisi parmi la cellulose, le polypropylène, une combinaison de cellulose et de polypropylène, la laine de polyéthylène hydrophile imprimée, le polyester et le polyéthylène.
